# EUROPEAN PATENT SPECIFICATION

(11) **EP 2 515 645 B1**
(45) Date of publication and mention of the grant of the patent: **07.02.2018**
(21) Application number: 10843596.7
(22) Date of filing: 22.12.2010
(51) Int. Cl.: A01N 25/34, A61K 8/00, A61K 8/18, A61K 9/14, A61K 9/16

(54) **ANTIFUNGAL THERAPY**
ANTIMYKOTISCHE THERAPIE
THERAPIE ANTIFONGIQUE

(30) Priority: 22.12.2009 US 289321 P; 06.08.2010 US 371604 P
(43) Date of publication of application: 31.10.2012
(73) Proprietor: Hallux, Inc., Santa Ana, CA 92704 (US)
(72) Inventor: BRIGHT, Corinne, Woodside CA 94062 (US); WAN, Jinping, Sunnyvale CA 94085 (US); BABLER, Martin, San Francisco CA 94131 (US); ZAMBOGLU, Ayse, Sunnyvale CA 94085 (US)
(74) Representative: J A Kemp
(86) International application number: PCT/US2010/061922
(87) International publication number: WO 2011/087867

(56) References cited:
- WO-A2-2006/063350
- CA-A1- 2 595 620
- US-A1- 2008 132 442
- US-A1- 2008 261 986
- US-A1- 2008 299 165
- US-A1- 2008 317 844

## Description

### FIELD

Compositions for use in treating onychomycosis by subungual implantation of the solid composition into a subungual nail bed in a region bound proximally by the lunula, laterally by the lateral nail folds and distally by the distal tip of the digit, are described herein. The compositions may be configured for local sustained release and include a biodegradable substrate and a release modifier.

### BACKGROUND

There are a variety of conditions that can affect the human nail. The pathophysiology of each condition is closely tied to nail structure and function. Thus, an understanding of nail anatomy and function is necessary in developing therapy for nail conditions.

In brief, the human nail is a modified cutaneous structure often described as a unit comprising several parts including, but not limited to, the nail matrix, the nail bed, the nail plate, the nail folds, the hyponychium, and the cuticle. The nail plate (fingernail or toenail) is produced by the matrix and progresses toward the tip of the fingers or toes as new plate is formed. The primary function of the nail plate is to protect the underlying digit, but fingernails and toenails are often also cosmetically important for many patients. The cutaneous tissue framing the nail unit, and which invaginates proximal and lateral to the nail plate, is referred to as the nail folds. The nail matrix is located beneath the proximal nail fold, and is the germinative portion of the nail unit that produces the nail plate. The lunula is the whitish crescent-shaped base of the nail and is the visible part of the nail matrix. The eponychium (or cuticle) is an outgrowth of the proximal fold, situated between the skin of the digit and the proximal end of the nail plate, fusing these structures together.
The hyponychium is epithelial tissue located beneath the distal end of the nail plate at the junction between the free edge of the nail plate and the skin of the fingertip. It forms a seal that protects the nail bed. The nail bed is the layer of tissue underneath the nail between the lunula and the hyponychium.

Various conditions can affect the nail unit, which includes the nail plate, nail bed, nail matrix, nail folds, and cuticle, individually or in combination, and the tissues adjacent to those structures in the distal phalanx. For example, the nail unit may be afflicted by inflammatory conditions such as psoriasis and lichen planus; nail tumors such as glomus tumor or digital myxoid cyst; and infections such as paronychia and onychomycosis. Onychomycosis is a common fungal infection of the nail bed, matrix, and/or nail plate. The primary clinical features of onychomycosis include distal onycholysis (separation of the nail plate from the nail bed), subungual hyperkeratosis, and a dystrophic, discolored nail. The fungal infection may be caused by dermatophytes (e.g., *Trichophyton rubrum* and T. mentagrophytes), but may also be due to infection by Candida species or nondermatophyte molds such as Aspergillus species, Scopulariosis brevicaulis, Fusarium species, and Scytalidium species.

Fungal infections of the nail are notoriously difficult to treat. Conventional topical therapies are typically unable to penetrate the nail plate, and thus eradicate the infection in the target tissue. Topical therapy accompanied by chemical or physical abrasion of the nails has also been largely unsuccessful. Given that topical antifungal therapy usually involves daily application to the nails for several months, patient compliance with such an extended treatment regimen is often problematic.

Oral antifungal agents may also be used to treat onychomycosis. For example, Nizoral® tablets (ketoconazole), Sporonox® capsules (itraconazole) (Janssen, Division of Ortho-McNeil-Janseen Pharmaceuticals, Inc., Titusville, NJ), Lamisil® tablets (terbinafine hydrochloride) (Novartis Pharmaceuticals, East Hanover, NJ), Diflucan® tablets (fluconazole) (Pfizer, New York, NY), and Grisfulvin V (griseofulvin) may be prescribed. However, systemic antifungal therapies are associated with potentially serious side effects such as heart and liver failure. The prolonged treatment regimens associated with oral antifungal therapy also usually result in poor patient compliance.

Relevant prior art documents are WO 2006/063350 A2 and CA2595620 A1.

Accordingly, alternative compositions for treating onychomycosis would be useful. Therapy associated with minimal side-effects would be desirable. Administration regimens having improved efficacy, and which are capable of individualizing onychomycosis treatment would also be desirable.

### SUMMARY

Described here are various solid compositions for use in treating onychomycosis by subungual implantation of the solid composition into a subungual nail bed in a region bound proximally by the lunula, laterally by the lateral nail folds and distally by the distal tip of the digit. It should be understood that the terms "composition" and "implant" are used interchangeably throughout. The compositions may be beneficial due to the particular release kinetics associated with them. For example, the compositions may include a release modifier that enhances or delays release of the active agent. Having the ability to manipulate active agent release may improve therapeutic efficacy.

For example, providing a bolus of the active agent to the nail unit, and more particularly the nail bed and nail plate, may provide a cidal effect in vivo. In some instances, providing levels of an antifungal agent to the nail bed at concentrations in excess of what can be achieved during or after oral therapy, may achieve a local fungicidal effect in a manner that cannot be obtained with conventional systemic therapy. This therapeutic regimen for treating, e.g., onychomycosis, may not only avoid the systemic side effects of oral therapy, but potentially allow for a shorter course of local therapy by significantly reducing or eliminating the fungi in the nail unit in the first few days of treatment. These higher concentrations in the first few days of administration may be beneficial to use in subjects with nail unit conditions in a mild to moderate state. Accordingly, the bolus or burst of drug released initially from the compositions described herein is generally configured to release drug within the therapeutic window. In the case of an antifungal agent, the composition may initially release enough drug to exert a cidal effect but not such a high level that local toxicity is observed. Signs of local toxicity may include erythema and edema in and around the implantation site.

Likewise, compositions having retarded, delayed, or pulsatile release may be beneficial to use in subjects with more severe nail unit conditions or with a higher disease relapse/recurrence rate, which may require a longer duration of therapy. The retarded or delayed release profiles may provide concentrations at or above those achieved by oral therapy in the target tissues after a single administration of the composition without a significant burst component. The specific administration regimen, location of implantation, and/or process of making the compositions may also be beneficial. The combination of one or more aspects of composition form, active agent release, administration regimen, implantation location, etc., may allow therapy to be individualized or optimized.

The solid compositions are for use in treating onychomycosis by subungual implantation of the solid composition into a subungual nail bed in a region bound proximally by the lunula, laterally by the lateral nail folds and distally by the distal tip of the digit. As used herein, the term "nail unit" refers to the nail matrix, nail plate, nail bed, nail folds, and cuticle, in combination, and the tissues adjacent to those structures in the distal phalanx. Examples of such adjacent tissues include epidermal tissue, dermal tissue, subcutaneous tissue (including adipose tissue), muscle, tendon, and bone in the region of the digit from the distal interphalangeal joint (or the distal-most interphalangeal joint) to the distal end of the tip of the digit, e.g., the distal end of the fingertip. As used herein, the term "nail unit condition" refers to a medical or cosmetic condition affecting any part of the nail unit and adjacent tissues. Furthermore, as used herein, the term "treat", "treating", or "treatment" refers to the resolution or reduction of symptoms or the underlying cause of the nail unit condition, or prevention of a nail condition. The terms "nail" or "nail plate" are herein used interchangeably throughout, and refer to fingernails or toenails.

The solid compositions are for use for use in treating onychomycosis by subungual implantation of the solid composition into a subungual nail bed in a region bound proximally by the lunula, laterally by the lateral nail folds and distally by the distal tip of the digit. Once administered, the compositions may release the active agent for treating onychomycosis over time periods of less than one week, at least about one week, at least about two weeks, at least about four weeks, at least about eight weeks, or at least about twelve weeks or more.

The compositions may be formulated to have a high drug load and may be configured for sustained release or immediate release of the active agent. For example, the compositions may include greater than about 30%, greater than about 40%, greater than about 50%, greater than about 60%, greater than about 70%, or greater than about 80% of the active agent by weight. The compositions are solid, that allows for placement by subungual implantation of the solid composition into a subungual nail bed in a region bound proximally by the lunula, laterally by the lateral nail folds and distally by the distal tip of the digit. Solid formulations may be of any suitable form e.g. the solid drug delivery systems may be formed as particles, sheets, discs, filaments, rods, and the like. Particle formulations include such forms as granules, pellets, beads crystals, microcapsules, nanoparticles, and microspheres. The compositions may comprise one or more active agents, carriers, excipents, and/or release modifiers, etc. If a carrier is included, the choice of carrier will usually depend on such factors as the form of system, specific active agent used, and the intended duration of treatment. However, in all instances the carrier will be biocompatible. In one variation, the carrier is biodegradable. In another variation, the carrier is bioerodible. In yet another variation, the carrier is bioabsorbable. As used herein, the term "biocompatible" refers to a carrier or matrix material that does not cause significant tissue irritation at the target site. The term "biodegradable" refers to carrier or matrix material that degrades over time by enzymatic or hydrolytic action, or other mechanism at the target site. By "bioerodible," it is meant that the carrier or matrix material erodes or degrades over time by contact with surrounding tissue fluids, through cellular activity or other physiological degradation mechanisms. By "bioabsorbable," it is meant that the carrier or matrix material breaks down and is absorbed by a cell, tissue, or other physiologic mechanism.

The compositions described herein may be sustained release microsphere compositions. The sustained release microsphere compositions may comprise an active agent, a biodegradable polymer, and between about 1% to about 10% by weight of a release modifier that enhances release of the active agent during the first few days of release. Here the composition may have an in vitro cumulative release profile in which greater than 5% of the active agent is released after about one day, greater than about 10% of the active agent is released after about 7 days, and greater than about 15% is released after about 12 days.

In some variations, the compositions are sustained release microsphere compositions including an active agent, a biodegradable polymer, and less than about 1% by weight of a release modifier that delays or retards release of the active agent during the first few days of release. Here, the composition may have an in vitro cumulative release profile in which less than 5% is released after about one day, less than 10% is released after about five days, and less than about 15% is released after about 10 days.

The compositions described herein generally include any suitable active agent suitable for treating onychomycosis. For example, an antifungal agent may be used. Exemplary antifungal agents that may be used include without limitation, amorolfine, ciclopirox, flucytosine, griseofulvin, haloprogrin, potassium iodide sodium pyrithione, undecylenic acid, imidazole derivatives, triazole derivatives, allylamines, polyene antifungal antibiotics, antifungal organic acids, and combinations thereof. Allylamines such as terbinafine may be especially beneficial.

The release modifiers may be hydrophilic surfactants. Exemplary hydrophilic surfactants that may be employed include without limitation, polyoxyethylene sorbitan fatty acid esters; polyoxyethylene-polyoxypropylene block copolymers; polyglycerol fatty acid esters; polyoxyethylene glycerides; polyoxyethylene sterols, derivatives, and analogues thereof; polyoxyethylene vegetable oils; polyoxyethylene hydrogenated vegetable oils; tocopheryl polyethylene glycol succinates; sugar esters; sugar ethers; sucroglycerides, and mixtures thereof. In one variation, the hydrophilic surfactant is a tocopheryl polyethylene glycol succinate, e.g., D-alpha-tocopheryl PEG-1000 succinate (vitamin E TPGS).

When a carrier or matrix forming material is included in the compositions, it may comprise a biodegradable, bioerodible or bioabsorbable material. The carrier may be a polymer and may comprise without limitation, natural and modified polysaccharides, proteins, biocompatible water-soluble polymers; natural and modified biodegradable polymers and synthetic biodegradable. In one variation, the composition comprises about 25% polyethylene glycol as the matrix forming material. In other variations, poly(lactic acid-co-glycolic acid) (PLGA) is used. PLGA is biocompatible and degrades by hydrolytic cleavage into nontoxic molecules that are easily eliminated from the body (namely, lactic acid and glycolic acid).

Other biodegradable polymers that may be used in the compositions include without limitation, alginate, cellulose, collagen, dextran, elastin, fibrin, polysaccharides, hyaluronic acid, polyacetal, polyacrylates (L-tyrosine-derived or free acid), poly(β-hydroxyesters), polyamides, poly(amino acid), polyalkanotes, polyalkylene alkylates, polyalkylene oxylates, polyalkylene succinates, polyanhydrides, polyanhydride esters, polyaspartimic acid, polylactic acid, polybutylene digloclate, poly(caprolactone), poly(caprolactone)/poly(ethylene glycol) copolymers, polycarbone, L-tyrosin-derived polycarbonates, polycyanoacrylates, polydihydropyrans, poly(dioxanone), poly-p-dioxanone, poly(ε-caprolactone-dimethyltrimethylene carbonate), poly(esteramide), polyesters, aliphatic polyesters, poly(etherester), polyethylene glycol/poly(orthoester) copolymers, poly(glutarunic acid), poly(glycolic acid), poly(glycolide), poly(glycolide)/poly(ethylene glycol) copolymers, poly(lactide), poly(lactide-co-caprolactone), poly(DL-lactide-co-glycolide), poly(lactide-co-glycolide)/poly(ethylene glycol) copolymers, poly(lactide)poly(ethylene glycol) copolymers, polypeptides, polyphosphazenes, polyphosphesters, polyphophoester urethanes, poly(propylene fumarate-co-ethylene glycol), poly(trimethylene carbone), polytyrosine carbonate, polyurethane, PorLastin or silk-elastin polymers, spider silk, tephaflex, terpolymer (copolymers of glycolide lactide or dimethyltrimethylene carbonate), and combinations, mixtures or copolymers thereof. Poly(lactic acid-co-glycolic acid) (PLGA) copolymers may be beneficial.

Subungual implantation of the solid composition occurs into a subungual nail bed in a region bound proximally by the lunula, laterally by the lateral nail folds and distally by the distal tip of the digit.

The placement location of the solid composition is beneficial in achieving high concentrations of active agent in the nail bed. For instance, as described in Examples 7 and 8, a local pharmacokinetic study evaluated the concentration of terbinafine in the distal nail bed after placing implants in the proximal nail fold, lateral nail fold, distal pulp (between the hyponychium and up to approximately 5 mm below the hyponychium), and nail bed (also referred to as subungual).

It was found that the location of placement of implants had a significant impact on the concentration of terbinafine in the nail bed. Implants placed in the subungual location showed the highest concentration of terbinafine in the nail bed among all implant sites. Further, comparison of results from implants placed in the distal pulp, lateral nail fold or proximal nail fold showed that implants placed in the distal pulp provided concentrations of terbinafine in the nail bed from 57 to up to more than 600 times higher than were found with the implants placed in the lateral or proximal nail folds.

Some variations of the method include implantation to achieve therapeutic concentrations in the nail unit according to a continuous regimen. When a continuous regimen is used, the regimen may involve implanting one or more sustained release compositions at three 30-day intervals, where the active agent is continuously released from the one or more compositions during each 30-day interval to provide 90 days of therapy. In some variations, a continuous regimen involves implanting one or more sustained release compositions at two 90-day intervals, where the active agent release is for 90-days to provide six months of therapy. In yet another variation, a continuous regimen involves implanting one or more sustained release compositions at three six-week intervals, where the active agent is released for 90 days to provide a total of four and a half months of therapy.

Other variations of the method include implantation according to a pulsed regimen. When a pulsed regimen is employed, the regimen may involve implanting one or more sustained release compositions at three 30-day intervals, where the active agent is released for two weeks of each 30-day interval. Some pulsed regimens may involve implanting one or more sustained release compositions at three 90-day intervals, where the active agent is released for three weeks of each 90-day interval. In some variations, the pulsed regimen may include implanting one or more sustained release compositions at 8 two-week intervals, where the active agent is released for one week of each two-week interval. In other variations, the pulsed regimen may include implanting one or more sustained release compositions at three 60-day intervals, where the active agent is released for one month of each 60-day interval. The pulsed regimens may also be designed to have one or more non-treatment intervals. For example, the non-treatment intervals may be at least about one week, at least about two weeks, or at least about three weeks, or at least about four weeks. In some instances, the non-treatment interval may be longer than four weeks.

For some patients with more severe disease, additional booster therapy may be necessary to fully cure the disease (after a course of continuous or pulsed therapy as previously described). For example, a physician evaluating the patient who has seen some initial clinical response may determine that the response is not being maintained in the patient, and thus may decide to implant a booster dose of therapy. In one variation, the additional booster treatment includes implanting one or more sustained release compositions at about 160 days after the end of the previous course of therapy. In another variation, the physician may elect to provide another entire course of therapy.

Described here are also various regimens for administering one or more antifungal compositions for the treatment or prophylaxis of onychomycosis. The solid antifungal compositions are for use in treating onychomycosis by subungual implantation of the solid composition into a subungual nail bed in a region bound proximally by the lunula, laterally by the lateral nail folds and distally by the distal tip of the digit. As further described below, the frequency of implantation and duration of antifungal agent release may be selected to optimize therapy for individual patients. The methods may also involve treating onychomycosis by any of the regimens noted above. The implantation interval here may also include intervals of 14 days, 30 days, 45 days, 60 days, three months, six months, or one year. Furthermore, in some variations of this method, one or more sustained release implants may be implanted for prophylaxis. For example, prophylaxis may be initiated about six months after a successful course of therapy. Alternatively, prophylaxis may be provided for one or more years to keep the nail unit free of disease.

### BRIEF DESCRIPTION OF THE DRAWINGS

FIG. 1 shows the cumulative in vitro release of an exemplary microsphere composition containing about 3000 µg of terbinafine over a 12 day period. Here terbinafine release is enhanced during the first few days of release.
FIG. 2 shows the cumulative in vitro release of another exemplary microsphere composition containing about 4000 µg of terbinafine over an 11 day period. Here terbinafine release is retarded during the first few days of release.
FIG. 3 shows the effect on the in vitro cumulative release of terbinafine from a further exemplary terbinafine microsphere composition when mannitol is added to the microsphere preparation process.
FIG. 4 shows the effect on the in vitro cumulative release of terbinafine from another exemplary terbinafine microsphere composition when sodium chloride is added to the microsphere preparation process.
FIGS. 5A-5I show exemplary locations of implantation within the nail unit and tissues adjacent the nail unit.

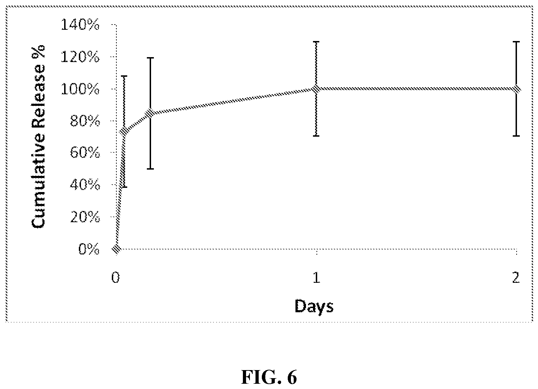

FIG. 6 shows the in vitro cumulative release of terbinafine from an solution over two days.
FIG. 7 depicts the in vitro cumulative release of terbinafine over 40 days from an exemplary microsphere composition made by a process in which vitamin E TPGS was added in the continuous phase.
FIG. 8 depicts an exemplary location within a digit for implantation of a composition within the distal pulp (area circumscribed by the lines).
FIG.9 depicts an exemplary location within a digit for implantation of a composition into the nail bed (subungual implantation) (area circumscribed by the lines).
FIG. 10 depicts exemplary combined locations within a digit for implantation of a composition within a digit that provide a high drug concentration in the nail bed (area circumscribed by the lines).

### DETAILED DESCRIPTION

Described here solid compositions for use in treating onychomycosis by subungual implantation of the solid composition into a subungual nail bed in a region bound proximally by the lunula, laterally by the lateral nail folds and distally by the distal tip of the digit. The compositions may be beneficial due to the particular release kinetics associated with them. For example, the compositions may include a release modifier that enhances or delays release of the active agent. As previously stated, having the ability to manipulate active agent release in this manner may improve therapeutic efficacy. For example, providing a bolus of the active agent in the first few days of administration may be beneficial to use in subjects with nail unit conditions in a mild to moderate state. Likewise, compositions having retarded or delayed release may be beneficial to use in subjects with more severe nail unit conditions. Combinations of bolus delivery and delayed release may also be used to treat the most severe nail unit conditions in which the entire nail unit is affected, including the proximal matrix.

The compositions may also be beneficial due to the particular administration regimen, location of implantation, specific type of composition and/or material properties of the composition. For example, as described further below, the efficiency of delivery was unexpectedly found to be dependant on the location of placement of the implant within the nail unit.

In the case of terbinafine delivering compositions, depending on the placement location, terbinafine levels in the region including the nail and tissues immediately adjacent the nail bed ranged from concentrations comparable to those found with oral dosing of terbinafine to up to approximately eleven-thousand times higher than orally dosed terbinafine. These concentrations are one hundred to over a million times higher than the minimum inhibitory concentration/minimum fungicidal concentration for terbinafine against the dermatophytes Trichophyton rubrum and Trichophyton mentragrophytes. It was further found that the concentration of terbinafine in these areas remained elevated for a significant period long after the implant had fully released the terbinafine contained in the implant. The combination of one or more aspects of composition form, active agent release, administration regimen, implantation location, etc., may allow therapy to be individualized or optimized.

### I. COMPOSITIONS

The solid compositions described here generally include an active agent and a biocompatible carrier or a matrix forming material that may be a biodegradable, bioerodible, or bioabsorbable polymer. The compositions may have any suitable form, and any suitable type of release, e.g., they may be configured for sustained release or immediate release. The solid composition may be, e.g., a cylindrical implant. The particles may be formed as granules, pellets, beads, microcapsules, and microspheres, and the like.

The compositions described here may be delivered in any size, shape, and/or volume compatible with the site of implantation, as long as they have the desired drug loading and release kinetics, and deliver an amount of active agent that is therapeutic for the intended nail condition. For example, the solid compositions may be formed as particles, sheets, discs, filaments, rods, and the like. The solid compositions may be formed to have volumes between 0 mm³ to about 20 mm³, between 0 mm³ to about 10 mm³, or between about 1 mm³ to about 20 mm³. In some instances, the solid compositions may be formed to have a volume between 0 mm³ to about 1 mm³. However, in some variations, the volume may be greater than 20 mm³.

In one variation, the composition is formulated as a solid implant and includes an active agent generally dispersed in a biocompatible carrier or matrix material. The carrier or matrix material may be any biocompatible polymeric or nonpolymeric material. The biocompatible materials may also be biodegradable, bioerodible, or bioabsorbable. The solid compositions include between 30-90% by weight of acitve agent.

The compositions described herein may also include particle compositions, e.g., microsphere compositions. The particle compositions may comprise an active agent, a solid phase material and optionally one or more excipents or other components such as one or more release modifiers. A particle composition generally includes a plurality of particles that typically have diameters of about between 0.1 µm to about 100 µm and preferably between about 1 µm to about 20 µm. The particle compositions may provide for immediate or sustained release of the active agent. Typically, when the particle compositions are intended for sustained delivery, the solid-phase material is a biocompatible polymer that provides sustained release of the active agent from the particle composition. The particles can have spherical, non-spherical or irregular shapes.

Sustained release compositions may also contain between about 0% to about 10% by weight of a release modifier that enhances release of the active agent during the first few days of release. Here the composition may have an in vitro cumulative release profile in which greater than 5% of the active agent is released after about one day, greater than about 10% of the active agent is released after about 7 days, and greater than about 15% is released after about 12 days. In another variation, the sustained release microsphere compositions are comprised of an active agent, a biodegradable polymer, and about between 0% to about 10% by weight of a release modifier that delays or retards release of the active agent during the first few days of release. Here, the composition may have an in vitro cumulative release profile in which less than 5% is released after about one day, less than 10% is released after about five days, and less than about 15% is released after about 10 days. In some variations, the compositions further include a release modifier that either enhances or retards release of the active agent.

### Active Agents

The active agents that may be used in the compositions described here include, but are not limited to, analgesics (narcotic and non-narcotic analgesics), anesthetics, anti-infective agents, anti-inflammatory agents, chemotherapeutic agents, other small molecules, and combinations thereof. Anti-infective agents generally include antibacterial agents, antifungal agents, antiviral agents, and antiseptics. Examples of anti-inflammatory agents include nonsteroidal anti-inflammatory agents and steroidal anti-inflammatory agents. Examples of chemotherapeutic agents include alkaloids, alkylating agents, antineoplastic antibiotics, and antimetabolites. Nucleic acids, peptides, and proteins are other classes of active agents that may be used.

The compositions may contain any suitable antifungal agent. Exemplary antifungal agents that may be used include, but are not limited to, ciclopirox; flucytosine; griseofulvin; haloprogrin; potassium iodide sodium pyrithione; pentamidine; dapsone; atovaquone; ; imidazole and triazole derivatives, including without limitation, albaconazole, bifonazole, butoconazole, clomidazole, clotrimazole, croconazole, econazole, fenticonazole, fluconazole, fosfluconazole, ketoconazole, isoconazole, luliconazole, miconazole, neticonazole, oxiconazole, sertaconazole,sulconazole and tioconazole; triazoles such as itraconazole, fluconazole, albaconazole; ravuconazole, sertaconazole, posaconazole, pramiconazole,terconazole, thiabendazole and voriconazole; allylamines, including without limitation, amorolofine, naftifine, butenafine, terbinafine; terbinafine FB, polyene antifungal antibiotics such as amphotericin B, candicin, filipin, natamycin, nystatin, and rimocidin; antifungal organic acids such as benzoic acid, borinic acid ester, salicylic acid, propionic acid, caprylic acid and undecylenic acid; selenium sulfide, tolnaftate, echinocandins such as abafungin, anidulafungin, caspofungin, and micafungin; tea tree oil, citronella oil, lemon grass, orange oil, patchouli, lemon myrtle, and Whitfield's ointment, and salts, free base forms, derivatives, analogs, and combinations thereof. In some variations, a combination of an antifungal agent and a steroidal anti-inflammatory agent are included. For example, corticosteroids (steroidal anti-inflammatory agents) including without limitation triamcinolone acetonide, dexamethasone, and betamethasone may also be co-administered in the composition with the antifungal agent.

The active agent, e.g., an antifungal agent, may constitute from about 10% to about 100% of the composition by weight. For example, the active agent may comprise between about 10% to about 90%, between about 10% to about 80%, between about 10% to about 70%, between about 10% to about 60%, between about 10% to about 50%, between about 10% to about 40%, between about 10% to about 30%, or between about 10% to about 20% by weight of the composition In some variations, the active agent may comprise from about 20% to about 50% or from about 20% to about 40% by weight of the composition. In other variations, the composition includes about 30% by weight of the active agent. In further variations the active agent, e.g., an antifungal agent may comprise between about 30% to about 90%, between about 60% to about 80%, or between about 65% to about 75% by weight of the implant.

The amount of active agent delivered to the tissues of the nail unit in a given administration may be between about 10 µg and about 1 g, or between about 0.5 mg and about 500 mg. In some variations, between about 1 mg and 5 mg, between about 1 mg and 4 mg, between about 1 mg and about 3 mg, or between about 1 mg and about 2 mg are delivered. In further variations, the amount of active agent delivered to the nail unit is between about 2 mg and about 5 mg.

In other variations, the amount of active agent delivered to the tissues of the nail unit in a given administration may be between about 1 µg and about 1 g, or between about 5 µg and about 500 mg. In some instances, between about 100 µg and 5 mg, between about 100 µg and 4 mg, between about 100 µg and about 3 mg, or between about 100 µg and about 2 mg are delivered.

### Biodegradable Polymers

The compositions may include one or more suitable biocompatible carrier or matrix forming materials that may be a biodegradable, bioerodible, or bioabsorbable polymer.

Exemplary biodegradable, bioerodible, or bioabsorbable materials include without limitation, natural and modified polysaccharides such as chitosan, alginate, cellulose, dextran, hyaluronic acid carboxymethylcellulose, hydroxypropylmethylcellulose, and the like, proteins such as collagen, gelatin, elastin, fibrin, laminin and the like; biocompatible water-soluble polymers such as, polyethylene glycols, polyvinylpyrrolidones and the like; polyacetal, polyacrylates (L-tyrosine-derived or free acid), poly(**α**-hydroxyesters), polyamides, poly(amino acid), polyalkanotes, polyalkylene alkylates, polyalkylene oxylates, polyalkylene succinates, polyanhydrides, polyanhydride esters, polyaspartimic acid, polylactic acid, polybutylene digloclate, poly(caprolactone), poly(caprolactone)/poly(ethylene glycol) copolymers, polycarbone, L-tyrosin-derived polycarbonates, polycyanoacrylates, polydihydropyrans, poly(dioxanone), poly-p-dioxanone, poly(**ε** -caprolactone-dimethyltrimethylene carbonate), poly(esteramide), polyesters, aliphatic polyesters, poly(etherester), polyethylene glycol/poly(orthoester) copolymers, poly(glutarunic acid), poly(glycolic acid), poly(glycolide), poly(glycolide)/poly(ethylene glycol) copolymers, poly(lactide), poly(lactide-co-caprolactone), poly(DL-lactide-co-glycolide), poly(lactide-co-glycolide)/poly(ethylene glycol) copolymers, poly(lactide)poly(ethylene glycol) copolymers, polypeptides, polyphosphazenes, polyphosphesters, polyphophoester urethanes, poly(propylene fumarate-co-ethylene glycol), poly(trimethylene carbone), polytyrosine carbonate, polyurethane, PorLastin or silk-elastin polymers, spider silk, tephaflex, terpolymer (copolymers of glycolide lactide or dimethyltrimethylene carbonate), and combinations, mixtures or copolymers thereof. In one variation, the composition comprises about 25% polyethylene glycol as the matrix forming material. In other variations, PLGA is used. PLGA is biocompatible and degrades by hydrolytic cleavage into nontoxic molecules that are easily eliminated from the body (namely, lactic acid and glycolic acid).

Further, the terminal functionalities of a polymer can be modified. For example, polyesters may be blocked, unblocked or a blend of blocked and unblocked polymers. A blocked polyester typically has blocked carboxyl end groups. Generally, the blocking group is derived from the initiator of the polymerization and is typically an alkyl group. An unblocked polyester generally has free carboxyl end groups.

Acceptable molecular weights for polymers used here may be determined by accounting for factors such as the desired polymer degradation rate, physical properties such as mechanical strength and rate of dissolution of polymer in solvent. Typically, an acceptable range of molecular weights is between about 2,000 Daltons and about 8,000,000 Daltons. Acceptable weight ranges for polyesters may be between about 5,000 Daltons and about 70,000 Daltons or about 15,000 Daltons and about 50,000 Daltons.

The biocompatible carrier may comprise from about 0% to about 99% of the composition by weight. For example, the biocompatible carrier may comprise between about 0% to about 90%, between about 0% to about 80%, between about 0% to about 70%, between about 0% to about 60%, between about 0% to about 50%, between about 0% to about 40%, between about 0% to about 30%, or between about 0% to about 20% by weight of the composition In some variations, the biocompatible carrier may comprise from about 10% to about 50% or from about 10% to about 40% by weight of the composition. In other variations, the composition includes about 70% by weight of the biocompatible carrier. In further variations, the biocompatible carrier may comprise between about 10% to about 40%, between about 20% to about 30%, or about 25% by weight of the implant.

### Release Modifiers

The compositions described here may be configured for any type and duration of release. One or more release modifiers may be included in the compositions described here. Exemplary release modifiers are hydrophilic surfactants. An empirical parameter commonly used to characterize the relative hydrophilicity and lipophilicity of (non-ionic) amphiphilic compounds such as surfactants is the hydrophilic-lipophilic balance (the "HLB" value). Surfactants with lower HLB values are more lipophilic, and have greater solubility in oils, whereas surfactants with higher HLB values are more hydrophilic, and have greater solubility in aqueous solutions. Using HLB values as a rough guide, hydrophilic surfactants are generally considered to be those compounds having an HLB value greater than about 10, as well as anionic, cationic, or zwitterionic compounds for which the HLB scale is not generally applicable. Similarly, lipophilic surfactants are compounds having an HLB value less than about 10.

Exemplary hydrophilic surfactants that may be included in the compositions described here include, without limitation, polyoxyethylene sorbitan fatty acid esters; polyoxyethylene-polyoxypropylene block copolymers; polyglycerol fatty acid esters; polyoxyethylene glycerides; polyoxyethylene sterols, derivatives, and analogues thereof; polyoxyethylene vegetable oils; polyoxyethylene hydrogenated vegetable oils; tocopheryl polyethylene glycol succinates; sugar esters; sugar ethers; sucroglycerides, and mixtures thereof. In one variation, the hydrophilic surfactant is a tocopheryl polyethylene glycol succinate, e.g., D-alpha-tocopheryl PEG-1000 succinate (vitamin E TPGS).

Sugars may also be used as release modifiers. Exemplary sugars include without limitation, monosaccharides, e.g., glucose, fructose, galactose, xylose, and ribose; disaccharides, e.g., lactose and sucrose; polysaccharides, e.g., cellulose, chitin, chitosen, glycogen, starch; and sugar alcohols, e.g., mannitol, glycol, glycerol, erythritol, threitol, arabitol, xylitol, ribitol, and sorbital; and combinations thereof.

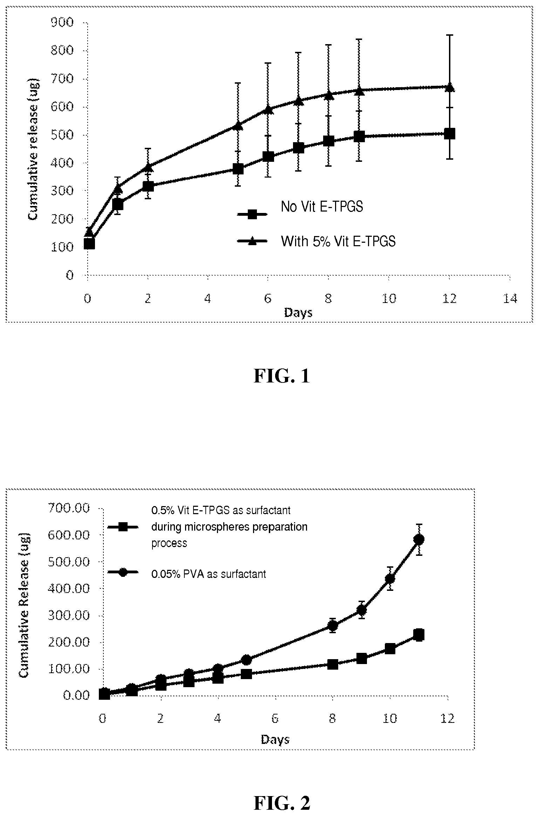

When a release modifier such as vitamin E TPGS is included in the compositions, release of the active agent may be enhanced or retarded depending on the amount used and the time of incorporation during the microsphere preparation process. For example, when about 5% vitamin E TPGS is used (see Example 2), the composition may be characterized as having the cumulative in vitro release profile of terbinafine shown in FIG. 1. Here release of terbinafine is enhanced during the first few days, being greater than about 5% after about one day, greater than about 10% after about 7 days, and greater than about 15% after about 12 days.

When about 0.5% or less vitamin E TPGS is included (see Example 3), the composition may be characterized as having the cumulative in vitro release profile of terbinafine shown in FIG. 2. Here release of terbinafine is retarded, being less than 5% after about one day, less than 10% after about five days, and less than about 15% after about 10 days.

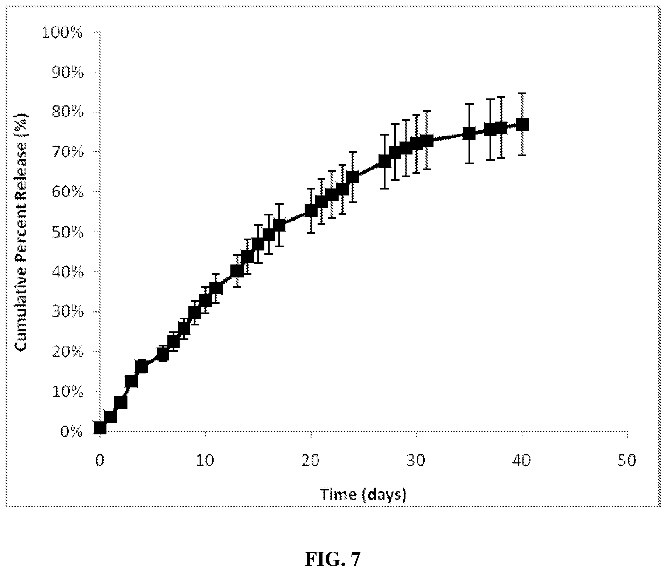

The release kinetics may also be varied depending on the conditions of manufacture. For example, as shown in FIG. 7, when vitamin E TPGS is added in the continuous water phase during the microsphere preparation process, release of the active agent, e.g., terbinafine, from the microsphere composition can occur over about 40 days.

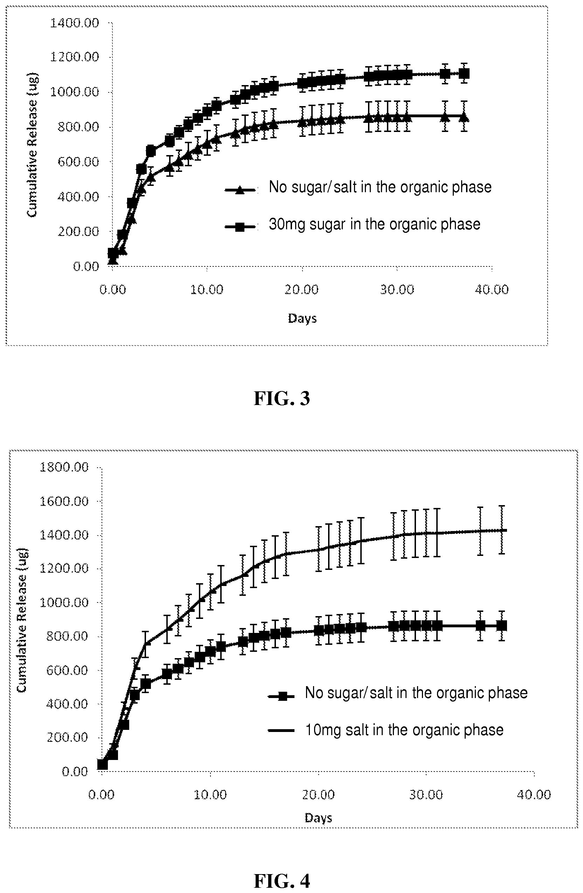

In further variations, a sugar alcohol, e.g., mannitol, may be included during the organic phase of microsphere preparation to modify active agent release. As shown in FIG. 3, such a prepared microsphere composition may result in an enhanced release profile where, e.g., release of the active agent from the composition may be about 150 µg at day 1, about 900 µg at day 7, and about 1250 µg at day 15. For comparison, microsphere compositions manufactured without the addition of sugar may release about 600 µg at day 7 and about 800 µg at day 15. This may occur because the addition of the sugar may create numerous channels in the polymer matrix of the microsphere, which facilitate diffusion of the active agent during dissolution testing. Other sugar alcohols that may be used include without limitation, glycol, glycerol, erythritol, threitol, arabitol, xylitol, ribitol, sorbital, and combinations thereof.

In other variations, a salt such as sodium chloride may be included in the compositions during the organic phase of microsphere preparation to modify active agent release. As shown in FIG. 4, such a prepared microsphere composition also results in an enhanced release profile where, e.g., release of the active agent from the composition may be about 180 µg at day 1, about 770 µg at day 7, and about 1000 µg at day 15. For comparison, microsphere compositions manufactured without the addition of salt may release about 100 µg at day 1, about 600 µg at day 7, and about 800 µg at day 15. As described above, this may occur because the addition of the salt may create numerous channels in the polymer matrix of the microsphere, which facilitate diffusion of the active agent during dissolution testing. The sugar and salts are insoluble in the organic solvent but soluble in water. Thus, during the second stage of microsphere preparation, when the primary emulsion is added into the continuous water phase, the sugar or salt dissolves in the water phase to thereby create the numerous channels.

### Other Additives

Other substances may be included in the compositions for a variety of purposes, including modification of active agent release. For example, buffering agents and preservatives may be employed. Preservatives which may be used include, but are not limited to, sodium bisulfite, sodium bisulfate, sodium thiosulfate, benzalkonium chloride, chlorobutanol, thimerosal, phenylmercuric acetate, phenylmercuric nitrate, ethyl paraben, methylparaben, polyvinyl alcohol and phenylethyl alcohol. Examples of buffering agents that may be employed include, but are not limited to, sodium carbonate, sodium borate, sodium phosphate, sodium acetate, sodium bicarbonate, and the like, as approved by the FDA for the desired route of administration. Electrolytes such as sodium chloride and potassium chloride may also be included in the compositions. Keratin softening agents such as alpha-hydroxy acids and urea may also be included. In some variations, one or more of the aforementioned additives is a release modifier.

The compositions may also include other components, such as a physiologically acceptable excipient or stabilizer. Some components can function both as a release modifier and as an excipent or stabilizer. A physiologically acceptable excipient, or stabilizer suitable for use in the composition is non-toxic to recipients at the dosages employed, and can include an antioxidant (e.g., ascorbic acid), a buffering agent (e.g., citrate), a low-molecular weight (e.g., less than about 20 residues) polypeptide, a protein (e.g., serum albumin), a hydrophilic or water-soluble polymer (e.g., polyethylene glycol or polyvinylpyrrolidone), an amino acid (e.g., glycine), a monosaccharide, a disaccharide, polysaccharide and other carbohydrates (e.g., including glucose, sucrose, mannose, dextrins, celluloses and carboxymethylcellulose), a chelating agent (e.g., ethylenediaminetetratacetic acid [EDTA]), a sugar alcohol (e.g., mannitol or sorbitol), a salt-forming counter ion (e.g., sodium), a metal cation (e.g., zinc), an anionic, non-ionic or cationic surfactant (e.g. Tween™ or Pluronics™,) and/or a preservative (e.g., propylparaben, methylparaben, quaternary ammonium salts, such as benzalkonium chloride).

As stated above, vitamin E TPGS may be used in the compositions to modify release of the active agent (here acting as a surfactant). However, it may also be included for a variety of other purposes. For example, vitamin E TPGS may be used in the compositions as a solubilizer, an emulsifier, a bio-availability enhancer, an anti-oxidant agent that prevents the propagation of free radical damage in biological membranes (because it is a potent peroxyl radical scavenger), a vehicle for a lipid-based drug formulation, or as a hot-melt extrusion aid.

### II. TERBINAFINE COMPOSITIONS

Variations of the composition may include microsphere compositions that specifically contain terbinafine as the active agent for use in treating onychomycosis by subungual implantation of the solid composition into a subungual nail bed in a region bound proximally by the lunula, laterally by the lateral nail folds and distally by the distal tip of the digit.

The microsphere compositions may generally include terbinafine HCL, PLGA as the biodegradable polymer, and vitamin E TPGS as the release modifier. When terbinafine release is to be enhanced during the first few days, about 1% to about 10% by weight of the vitamin E TPGS may be included in the compositions. In some variations, about 5% vitamin E TPGS is included. When terbinafine release is to be retarded, about 0.5% by weight or less of the vitamin E TPGS may be included in the compositions.

Solid compositions formed as implants may include terbinafine and a biocompatible matrix forming material. In one variation, the implant is rod shaped with a length of about 1mm to about 10 mm and a diameter of less than about 1mm. In another variation, the rod shaped implant has a length of about 4 mm and a diameter of about 0.4mm. The implants may comprise between about 30% to 90% terbinafine HCL and between about 10% and about 70% polyethylene glycol. In other variations, the implants may comprise about 75% terbinafine HCL and about 25% polyethylene glycol.

### III. METHODS

### Composition Delivery

The compositions described here are for use in treating onychomycosis by subungual implantation of the solid composition into a subungual nail bed in a region bound proximally by the lunula, laterally by the lateral nail folds and distally by the distal tip of the digit. Delivery into the digit may be accomplished using any suitable implantation device. For example, the applicator may be manually operated or automated. The applicator may also include a needle, trocar, or other sharp conduit, forceps, a pusher, a syringe, slide buttons, etc. The applicator described in commonly owned co-pending U.S. Application Serial No.61/263,207 may also be used to implant the compositions. In some variations, the applicators are preloaded with one or more compositions.

In general, the volume of the solid composition to be delivered will be less than about 100 µl, and in some instances, less than about 10 µl may be used. In some variations, volumes between greater than about 0 µl and about 5 µl may be employed. Given these small volumes, fine gauge needles will generally be used to deliver the compositions. For example, 19 gauge, 21 gauge, 23 gauge, 25 gauge, 26 gauge, 27 gauge, 28 gauge, 29 gauge, or 30 gauge needles may be used.

Prior to implantation, the skin overlying the area of implantation may be cleaned using a disinfectant wipe such as an alcohol wipe, and / or pre-treated with a local anesthetic. Local anesthetics that may be topically applied include without limitation, EMLA® anesthetic cream (AstraZeneca, Wilmington, DE) and Topicaine® anesthetic gel (ESBA Laboratories, Jupiter, FL). In some variations, a local anesthetic such as lidocaine (Xylocaine) with or without epinephrine bitartrate may be injected at the area of implantation prior to the implantation of the composition.

The solid compositions are for use in treating onychomycosis by subungual implantation of the solid composition into a subungual nail bed in a region bound proximally by the lunula, laterally by the lateral nail folds and distally by the distal tip of the digit. Any number of compositions may be implanted. The patient may have the compositions implanted during a physician visit.

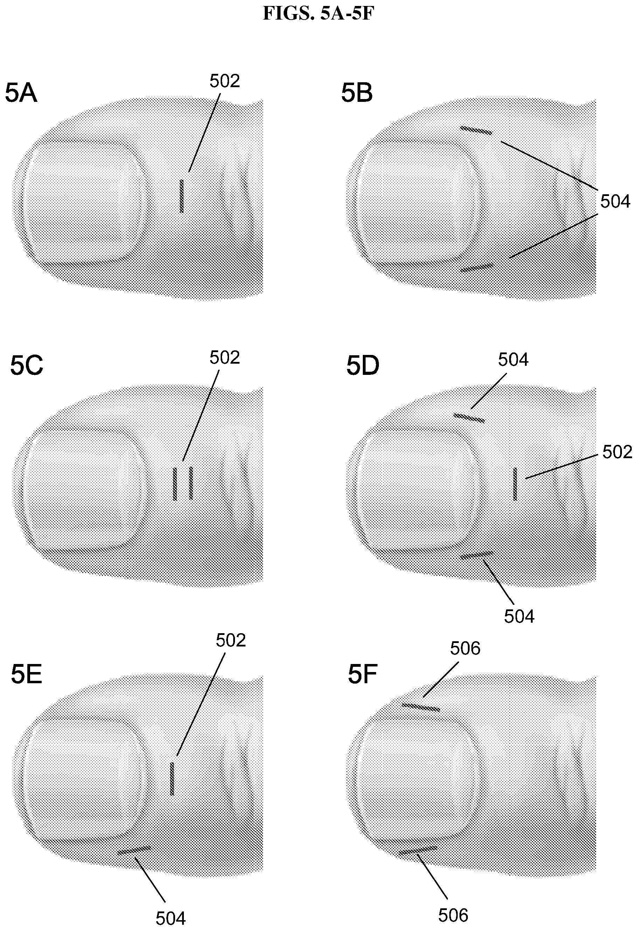

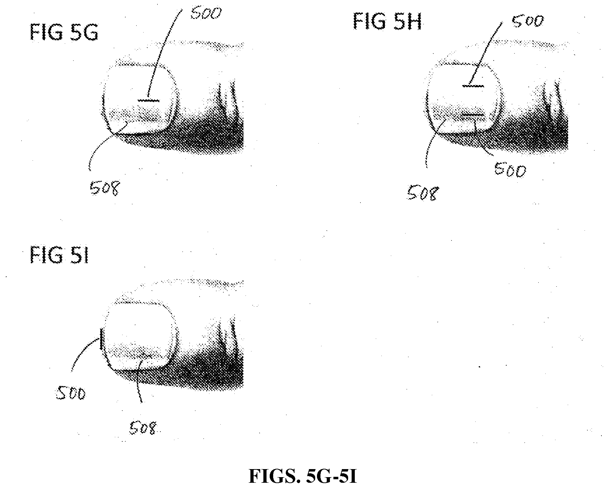

Non-exemplary locations are shown in FIGS. 5A-5F.

The compositions depicted in FIGS. 5G and 5H may be implanted by insertion of an implantation device through the skin of the distal tip of the digit and into the underlying tissue of the distal tip of the digit or the lateral nail fold and then travelling into the nail bed to implant the composition(s) at the locations shown. These compositions are generally implanted distal to the lunula so as not to disrupt the nail matrix.

More specifically, a user such as a dermatologist, podiatrist, general practitioner, internist, physician's assistant, or other healthcare provider may administer one or more solid implants, (e.g., approximately 3-6 mm long), containing terbinafine into any of the above described locations for the treatment of distal subungual onychomycosis. The implant is intended for being placed intradermally into the nail bed by subungual implantation of the solid composition into a subungual nail bed in a region bound proximally by the lunula, laterally by the lateral nail folds and distally by the distal tip of the digit, approximately 1-5 mm below the hyponychium using a 25-gauge needle. In this instance, the implant may be in the lumen of the needle and a wire piston at the opposite end of the needle may be used to expel the implant. The implantation may be repeated at appropriate intervals, e.g., weekly, once every two weeks, once per month, once every two months, or once every three months.

### Dosing

The implantations described above may be applied to the infected fingers or toes at monthly intervals for four or six months. Alternatively, a patient may receive more frequent doses initially, in an induction period, followed by a maintenance period thereafter at lees frequent intervals unitil cure.

As previously stated, for some patients with more severe disease, additional booster therapy may be necessary to fully cure the disease (after a course of continuous or pulsed therapy as previously described). For example, a physician evaluating the patient who has seen some initial clinical response may determine that the response is not being maintained in the patient, and thus may decide to implant a booster dose of therapy. In one variation, the additional booster treatment includes implanting one or more sustained release compositions at about 160 days after the end of the previous course of therapy. In another variation, the physician may elect to provide another entire course of therapy.

Patients with residual nail disease or signs of relapse or reinfection from a previous onychomycosis course of therapy may also be treated prophylactically.

In one variation, the method of implantation involves advancing a sharp conduit such as a needle through the skin and into a subungual nail bed in a region bound proximally by the lunula, laterally by the lateral nail folds and distally by the distal tip of the digit, The sharp conduit may have a depth marker that indicates the appropriate depth to which the conduit should be advanced. Once the tip of the sharp conduit is positioned at the target area (at or near the area affected by onychomycosis), one or more solid antifungal compositions (compositions including an antifungal agent) are then implanted. Any suitable method for delivering the composition from the applicator into the target area may be used. For example, a push rod, pressurized gas, mandrel, etc., may be used during the implantation process. In some instances, such components may be used to advance the implant from the applicator into the digit. In other instances, the components are used to maintain the position of implant while the sharp conduit is being advanced into the digit.

The compositions may be used according to any suitable administration regimen or protocol, and may depend on a number of factors, such as the severity and extent of the fungal infection, presence of any underlying medical conditions, and patient compliance with follow-up visits. In some instances, the administration regimens will mimic therapeutic regimens that employ oral antifungal dosage forms. The administration regimens described here may include implanting one or more compositions at 7-day intervals, 14-day intervals, 30-day intervals, 45-day intervals, 60-day intervals, three month intervals, four month intervals, six month intervals, or yearly intervals (implantation intervals) and may be called a pulsed therapy regimen. Any number of implantation intervals may also be employed. The total duration of treatment may be between one week and one year, although a patient may be treated prophylactically beyond this. For example, an administration regimen may include implanting one or more antifungal compositions on three occasions, with a 30 day interval between each occasion for a total duration of approximately two months. Alternatively, an administration regimen may include four administrations: including implanting one or more antifungal compositions on three occasions, with a 30 day interval between each occasion, followed by one more administration after a 60-day interval for a total duration of approximately four months. In another example, an administration regimen may include four administrations: implanting one or more antifungal compositions on four occasions, with a 90-day interval between each occasion, for a total of one year. This regimen may be extended with prophylactic treatment at 90-day intervals in order to keep the patient disease-free or to clear any minor residual nail disease (<10% nail involvement).

The compositions may be configured to deliver the antifungal agent for any suitable duration. For example, the compositions may be configured to deliver the antifungal agent, for at least about one week (seven days), at least about two weeks (14 days), at least about three weeks (21 days), at least about one month (30 days), at least about one and a half months (42 days), at least about two months (60 days), at least about three months (90 days), at least about six months, or at least about one year. In some variations, the active agent is delivered continuously from the composition. In other variations, the active agent is delivered in pulses from the composition.

When a pulsed therapy regimen is employed, any duration of antifungal agent delivery may be used. For example, a composition configured to deliver an antifungal agent for one week may be administered on four occasions at one month intervals. Here therapy the pulse therapy is provided for one week per month for three months. In another variation, the composition may be configured to deliver an antifungal agent for at least about two weeks, at least about three weeks, or at least about four weeks or more. In yet a further variation, a composition configured to deliver therapy for four weeks may be delivered at three-month intervals.

The duration of antifungal delivery may be combined with any non-treatment interval. For example, the non-treatment interval may be about one week, about two weeks, about three weeks, about four weeks, about five weeks, or about six weeks or more. In some variations, the pulse therapy regimen includes administering the active agent at an interval of every two weeks per month, and a non-treatment interval of two weeks per month. In other variations, the pulse therapy regimen includes administering the active agent for four weeks, and a non-treatment interval of two weeks. Whether or not a pulse therapy regimen is used, the ability to vary the type of compositions used, number of implants, implantation intervals, and non-treatment intervals will generally be able to provide customized/individualized therapy, e.g., onychomycosis therapy.

The total dose delivered for the active agent may vary depending on such factors as the particular agent used and whether the composition is being administered for treatment or prophylaxis. For example, the active agent delivered to the tissues of the nail unit in a given administration may be between about 10 µg and about 1 g, or between about 0.5 mg and about 500 mg. In some variations, between about 1 mg and 5 mg, between about 1 mg and 4 mg, between about 1 mg and about 3 mg, or between about 1 mg and about 2 mg are delivered. In further variations, the amount of active agent delivered to the nail unit is between about 2 mg and about 5 mg.

### IV. PHARMACOKINETICS

The release kinetics of the compositions described here may be due, in part, to the amount of the active agent loaded, the polymer or polymers used, the addition of any release modifiers, or the conditions of manufacture or a combination of these factors.

The placement of the into a subungual nail bed in a region bound proximally by the lunula, laterally by the lateral nail folds and distally by the distal tip of the digit, is beneficial in achieving high concentrations of active agent in the nail bed. For example, as further described in Examples 7 and 8, a local pharmacokinetic study in healthy volunteers was undertaken to evaluate the concentration of terbinafine in the distal nail bed after placing implants in the proximal nail fold, lateral nail fold, distal pulp (between the hyponychium and up to approximately 5 mm below the hyponychium), and subungual nail bed. The results obtained from the different implantation sites were compared to one another and to oral terbinafine therapy (250 mg/day). Implants were delivered through a 25-gauge needle to the given location on day 1 and 3-mm punch biopsies of the nail bed and nail plate were subsequently taken on days 4, 15, 29, and 43. Another group received systemic terbinafine therapy administered orally once per day (250 mg/day) until the day of the punch biopsy on days 8, 15, and 29.

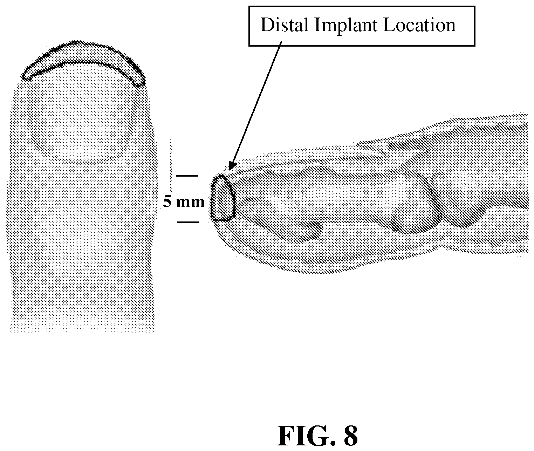

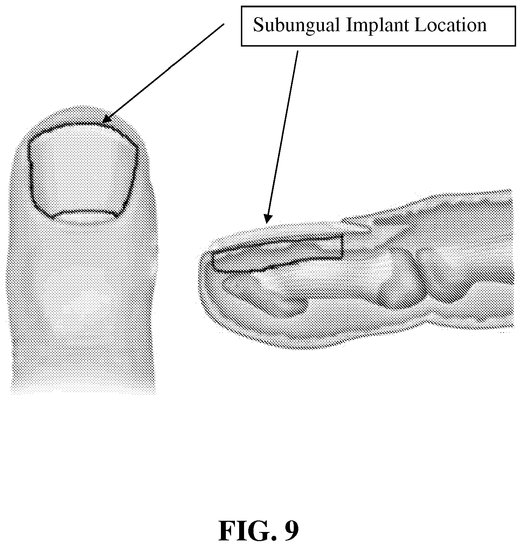

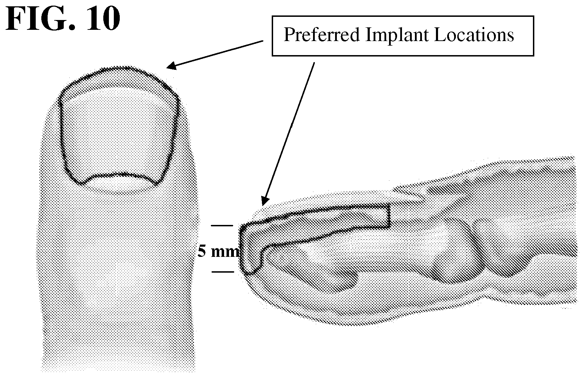

It was found that the location of placement of implants within the distal portion of the digit had a significant impact on concentration of terbinafine in the nail bed. Implants placed in the subungual location (e.g., the tissue below the hyponychium circumscribed by the lines shown in FIG. 9) showed the highest concentration of terbinafine in the nail bed among all implant sites. Further, comparison of results from implants placed in the distal pulp, lateral nail fold or proximal nail fold showed that implants placed in the distal pulp (e.g., the tissue below the hyponychium circumscribed by the lines shown in FIG. 8) provided concentrations of terbinafine in the nail bed from approximately 57 to up to approximately 600 times higher than were found with the implants placed in the lateral or proximal nail folds. These regions taken together encompass the region depicted in FIG. 10.

Terbinafine concentrations in the nail bed ranged from comparable to oral to up to approximately ten-thousand times higher than oral depending on the number of days after implantation and the implant site location. These concentrations are one hundred to over a million times higher than the minimum inhibitory concentration/minimum fungicidal concentration for terbinafine against the dermatophytes *Trichophyton rubrum and Trichophytom mentragrophytes.*

This application further discloses the following embodiments 1-24 for use in treating onychomycosis by subungual implantation of the solid composition into a subungual nail bed in a region bound proximally by the lunula, laterally by the lateral nail folds and distally by the distal tip of the digit:
Embodiment 1. A sustained release microsphere composition comprising an active agent, a biodegradable polymer, and between about 1% to about 10% by weight of a release modifier, wherein the composition has an in vitro cumulative release profile in which greater than 5% of the active agent is released after about one day, greater than about 10% of the active agent is released after about 7 days, and greater than about 15% is released from the microsphere composition after about 12 days.
Embodiment 2. The sustained release composition of embodiment 1, wherein the composition comprises between about 5% to about 10% by weight of a release modifier.
Embodiment 3. The sustained release composition of embodiment 1, wherein the composition comprises about 5% by weight of a release modifier.
Embodiment 4. The sustained release microsphere composition of embodiment 1, wherein the release modifier comprises a hydrophilic surfactant.
Embodiment 5. The sustained release microsphere composition of embodiment 4, wherein the hydrophilic surfactant is selected from the group consisting of polyoxyethylene sorbitan fatty acid esters; polyoxyethylene-polyoxypropylene block copolymers; polyglycerol fatty acid esters; polyoxyethylene glycerides; polyoxyethylene sterols, derivatives, and analogues thereof; polyoxyethylene vegetable oils; polyoxyethylene hydrogenated vegetable oils; tocopheryl polyethylene glycol succinates; sugar esters; sugar ethers; sucroglycerides, and mixtures thereof.
Embodiment 6. The sustained release microsphere composition of embodiment 5, wherein the hydrophilic surfactant comprises a tocopheryl polyethylene glycol succinate.
Embodiment 7. The sustained release microsphere composition of embodiment 6, wherein the tocopheryl polyethylene glycol succinate comprises D-alpha-tocopheryl PEG-1000 succinate (vitamin E TPGS).
Embodiment 8. The sustained release microsphere composition of embodiment 1, wherein the active agent comprises an antifungal agent.
Embodiment 9. The sustained release microsphere composition of embodiment 8, wherein the antifungal agent is selected from the group consisting of amorolfine, ciclopirox, flucytosine, griseofulvin, haloprogrin, potassium iodide sodium pyrithione, undecylenic acid, imidazole derivatives, triazole derivatives, allylamines, polyene antifungal antibiotics, antifungal organic acids, and combinations thereof.
Embodiment 10. The sustained release microsphere composition of embodiment 9, wherein the imidazole derivative is selected from the group consisting of bifonazole, butoconazole, clotrimazole, econazole, ketoconazole, miconazole, oxiconazole, and sulconazole.
Embodiment 11. The sustained release microsphere composition of embodiment 9, wherein the triazole derivative is selected from the group consisting of itraconazole, fluconazole, and terconazole.
Embodiment 12. The sustained release microsphere composition of embodiment 9, wherein the allylamine comprises naftifine or terbinafine.
Embodiment 13. The sustained release microsphere composition of embodiment 12, wherein the allylamine comprises terbinafine.
Embodiment 14. The sustained release microsphere composition of embodiment 9, wherein the polyene antifungal antibiotic comprises amphotericin B or nystatin.
Embodiment 15. The sustained release microsphere composition of embodiment 9, wherein the antifungal organic acid is selected from the group consisting of benzoic acid, salicylic acid, propionic acid, and caprylic acid.
Embodiment 16. The sustained release microsphere composition of embodiment 8, wherein the antifungal agent comprises about 10% to about 60% by weight of the composition.
Embodiment 17. The sustained release microsphere composition of embodiment 1, wherein the biodegradable polymer is selected from the group consisting of alginates, celluloses, collagen, dextran, elastin, fibrin, polysaccharides, hyaluronic acid, polyethylene glycols, polyacetal, polyacrylates (L-tyrosine-derived or free acid), poly(■-hydroxyesters), polyamides, poly(amino acid), polyalkanotes, polyalkylene alkylates, polyalkylene oxylates, polyalkylene succinates, polyanhydrides, polyanhydride esters, polyaspartimic acid, polylactic acid, polybutylene digloclate, poly(caprolactone), poly(caprolactone)/poly(ethylene glycol) copolymers, polycarbone, L-tyrosin-derived polycarbonates, polycyanoacrylates, polydihydropyrans, poly(dioxanone), poly-p-dioxanone, poly(■-caprolactone-dimethyltrimethylene carbonate), poly(esteramide), polyesters, aliphatic polyesters, poly(etherester), polyethylene glycol/poly(orthoester) copolymers, poly(glutarunic acid), poly(glycolic acid), poly(glycolide), poly(glycolide)/poly(ethylene glycol) copolymers, poly(lactide), poly(lactide-co-caprolactone), poly(DL-lactide-co-glycolide), poly(lactide-co-glycolide)/poly(ethylene glycol) copolymers, poly(lactide)poly(ethylene glycol) copolymers, polypeptides, polyphosphazenes, polyphosphesters, polyphophoester urethanes, poly(propylene fumarate-co-ethylene glycol), poly(trimethylene carbone), polytyrosine carbonate, polyurethane, PorLastin or silk-elastin polymers, spider silk, tephaflex, terpolymer (copolymers of glycolide lactide or dimethyltrimethylene carbonate), and combinations, mixtures or copolymers thereof.
Embodiment 18. The sustained release microsphere composition of embodiment 16, wherein the biodegradable polymer comprises a poly(lactic acid-co-glycolic acid) (PLGA) copolymer.
Embodiment 19. A sustained release microsphere composition comprising an active agent, a biodegradable polymer, and less than about 1% by weight of a release modifier, wherein the composition has an in vitro cumulative release profile in which less than 5% is released after about one day, less than 10% is released after about five days, and less than about 15% is released after about 10 days.
Embodiment 20. The sustained release microsphere composition of embodiment 19, wherein the composition comprises about 0.5% by weight or less of the release modifier.
Embodiment 21. The sustained release microsphere composition of embodiment 19, wherein the release modifier comprises vitamin E TPGS.
Embodiment 22. The sustained release microsphere composition of embodiment 19, wherein the active agent is an antifungal agent.
Embodiment 23. The sustained release microsphere composition of embodiment 22, wherein the antifungal agent comprises terbinafine.
Embodiment 24. The sustained release microsphere composition of embodiment 19, wherein the biodegradable polymer comprises a poly(lactic acid-co-glycolic acid) (PLGA) copolymer.

### V. EXAMPLES

The following examples are intended to be illustrative and not to be limiting.

### Example 1: Preparation of Terbinafine Loaded PLGA Microspheres

Terbinafine loaded PLGA microspheres were prepared by conventional emulsion solvent evaporation or spray drying methods. For the emulsion solvent evaporation method, a predetermined amount of terbinafine HCl (150mg) was dissolved in the oil phase (polymer in solvent 100mg/1000g). The polymer was 50/50 polylactic acid/glycolic acid with a molecular weight of about 25,000 g/mol. The solvent was methylene chloride. The aqueous phase (100g) contained the surfactant polyvinyl alcohol (0.5g) to adjust viscosity. The drug/polymer solution was vortexed or sonicated with the aqueous phase for 1 min, generating the first emulsion. The first emulsion was then added into a stirring continuous phase (a PVA aqueous solution) to evaporate the organic solvent. A few hours later, solidified microspheres were washed, collected, and dried.

For the spray drying method, 1g of terbinafine HCl and 1g of 50/50 polylactic acid/polyglycolic acid copolymer with the molecular weight of about 25,000 g/mol were dissolved in methylene chloride and sprayed through a micro sized nozzle. The generated particles were then solidified in a heated chamber at the temperature of 65ûC and collected in a collecting vessel.

### Example 2: Vitamin E TPGS Incorporated Into the PLGA Polymer Matrix (Release Enhancer)

Vitamin E TPGS incorporated PLGA microspheres were prepared via the emulsion solvent evaporation or the spray drying methods as described in Example 1. 1-10% w/w Vitamin E TPGS was then added into the drug polymer solution during the preparation process. It was found that vitamin E TPGS incorporated PLGA microspheres can increase the release rate of the terbinafine from the PLGA microspheres during the first few days of release (see FIG. 1).

### Example 3: Vitamin E TPGS as a Release Retarder

As previously stated, Vitamin E TPGS may also be used to retard terbinafine release from PLGA microspheres. During the emulsion solvent evaporation process, vitamin E TPGS was used as the surfactant to form a stable emulsion. However, after the microsphere preparation process, free vitamin E TPGS was then washed off, leaving only a small amount of the vitamin E TPGS attached onto the microsphere surface. As shown in FIG. 2, the surface attached vitamin E TPGS may function as a release modifier, but unlike the composition of Example 2, the vitamin E TPGS is a release retarder (not a release enhancer).

### Comparative example 4: Preparation of Aqueous Solution of Terbinafine in 0.1 % Tween 80 in Water

The solubility of terbinafine HCl in phosphate-buffered saline (PBS 1 X, starting pH 7.4) was 0.78 mg/mL and the final pH, 4.2. With 0.1% Tween-80 in water, the solubility of terbinafine HCl in terbinafine HCl-PEG 3350 blend (75/25, W/W) was 4 mg/mL. The resulting solution was clear but turned cloudy after standing at room temperature for 5 days. The final pH was 3.0.

### Comparative Example 5: Preparation of Nonaqueous Solution of Terbinafine in Dimethyl Sulfoxide

When DMSO is used as the solvent, the solubility of terbinafine HCl was close to 100 mg/mL. The solution was stable up to 7 days and dropped to 90 mg/mL at 14 days and remained constant up to 22 days. The solution was made by weighing 100 mg of terbinafine HCl powder in a glass vial and then adding 1 ml of DMSO solvent. The glass vial was sealed and vortexed for 5 minutes with a mini vortexor. The resulting solution was clear.

### Example 6: Terbinafine Extruded Compositions

A terbinafine extruded composition was made by first mixing terbinafine HCl and PEG at a ratio of 75:25 respectively (total weight of the mixture was 0.5 g). The mixture was filled into a batch extruder and heated for about one hour at 100°C. The melt was then extruded through a circular orifice to create a filament having a diameter of about 0.38 mm. From the filament, subunits of 4 mm in length were cut.

Terbinafine release from the implant was measured as follows. One implant made according to the method described above was placed into screw cap glass vials filled with 10 ml of phosphate buffered saline adjusted to pH 3 (PBS) and placed into a shaking water bath kept at a temperature of 37°C. At designated time points, the solution is decanted from the implants and replaced with the same amount of fresh pH 3 PBS. The samples are then analyzed for drug concentration by techniques known in the art, such as spectroscopy, HPLC, and the like. These implants have a drug release profile as shown in the following table:

| Time | 1 Hour | 2 Hour | 4 Hour | 6 Hour | 24 Hour |
|---|---|---|---|---|---|
| % release | 17.5 | 29.5 | 49 | 68.5 | 100 |

### Example 7: Pharmacokinetic Study of Terbinafine Extruded Drug Delivery System

Pharmacokinetics studies were conducted in humans subjects with a micro-implant made according to the method described in Example 6. Healthy volunteers were randomly assigned to the groups in Table 1. Subjects in Group 1 were instructed to take one 250 mg tablet of terbinafine HCl orally daily for 7 days. Subjects in Group 2 received one terbinafine micro-implant which was implanted into the nail bed tissue of the hallux, approximately 1 mm below the nail. Subjects in Group 3 received 1 terbinafine micro-implant which was implanted into the distal pulp of the hallux, approximately 1 to 5 mm below the hyponychium. On Day 8 for subjects in Group 1 and Day 4 for subjects in Groups 2 and 3, distal nail punch biopsies that included the nail and nail bed were obtained from each subject. For each biopsy sample, the nail bed tissue was separated from the nail plate and the nail bed tissues were analyzed for terbinafine. The average result for each group is presented in Table 1. Terbinafine concentrations in the tissue samples of Groups 2 and 3 were found to be significantly higher than those in the subjects receiving oral terbinafine HCl.

**Table 1**

| Group | Treatment | Implant Location | Biopsy Time Point | Number of Subjects | Average Concentration (Range) µg terbinafine / gm nail bed tissue |
|---|---|---|---|---|---|
| 1 | Oral terbinafine for 7 days | N/A | Day 8 | 4 | 0.4 (0.108 - 1.093) |
| 2 | 1 Implant on Day 1 | Subungual | Day 4 | 2 | 778.5 (447 - 1110) |
| 3 | 1 Implant on Day 1 | Distal Pulp | Day 4 | 3 | 19.5 (0.113 - 43.2) |

### Example 8: Pharmacokinetic study of Terbinafine Extruded Compositions

Pharmacokinetics studies were conducted in humans subjects with a micro-implant made according to the method described in Example 6. Healthy volunteers were randomly assigned to the groups in Table 2. Subjects in Group 1 received 3 terbinafine micro-implants which were implanted into the distal pulp of the hallux, approximately 1 to 5 mm below the hyponychium. Subjects in Group 2 received 3 terbinafine micro-implants which were implanted into the lateral nail fold of the hallux. Subjects in Group 3 received 3 terbinafine micro-implants which were implanted into the proximal nail fold of the hallux. On Day 4, distal nail punch biopsies that included the nail and nail bed were obtained from each subject. For each biopsy sample, the nail bed tissue was separated from the nail plate and the nail bed tissue was analyzed for terbinafine. The average result for each group is presented in Table 2. Terbinafine concentrations in the tissue samples from subjects in Group 1 (distal implants) were found to be significantly higher than those in the Group 2 and 3 subjects (proximal and lateral implants respectively).

**Table 2**

| Group | Treatment | Implant Location | Nail Punch Biopsy Time Point | Number of Subjects | Average Concentration (Range) µg terbinafine / gm tissue |
|---|---|---|---|---|---|
| 1 | 3 TMI-358 on Day 1 | Distal | Day 4 | 1 | 78.4 (N/A) |
| 2 | 3 TMI-358 on Day 1 | Proximal | Day 4 | 2 | 0.173 (0.124 - 0.221) |
| 3 | 3 TMI-358 on Day 1 | Lateral | Day 4 | 2 | 0.80 (0.238 - 1.368) |

## Claims

1. A solid composition for use in treating onychomycosis by subungual implantation of the solid composition into a subungual nail bed in a region bound proximally by the lunula, laterally by the lateral nail folds and distally by the distal tip of the digit, the solid composition comprising one or more sustained release solid polymer implants, the one or more sustained release solid polymer implants comprising between about 30-90% by weight of an active agent, said solid composition being subungually implanted by inserting an implantation device through skin or tissue at the distal tip of a digit according to a predetermined therapeutic regimen comprising predetermined implantation intervals.

2. The composition for use according to claim 1, wherein the implantation interval is selected from the group consisting of about 14 days, about 30 days, about 45 days, about 60 days, about three months, about six months, and about one year.

3. The composition for use according to claim 1 or claim 2, wherein the active agent comprises terbinafine.

4. The composition for use according to claim 1, wherein the one or more sustained release compositions comprise a biodegradable polymer.

5. The composition for use according to claim 4, wherein the biodegradable polymer comprises a poly(lactic acid-co-glycolic acid) (PLGA) copolymer.

6. The composition for use according to claim 4, wherein the biodegradable polymer comprises polyethylene glycol.

7. The composition for use according to claim 1, wherein the predetermined therapeutic regimen is a continuous regimen or a pulsed regimen.

8. The composition for use according to claim 7, wherein the pulsed regimen comprises one or more non-treatment intervals of at least two weeks.

9. Use of a solid composition for the manufacture of a medicament for use in treating an infection fo the nail unit by subungual implantation of the solid composition into a subungual nail bed in a region bound proximally by the lunula, laterally by the lateral nail folds and distally by the distal tip of the digit, the composition comprising one or more solid polymer compositions comprised of between about 30-90% by weight of an anti-infective agent.

10. The use according to claim 9, wherein the one or more compositions have a volume between 0.1 µl to 50 µl.

11. The use according to claim 9, wherein the anti-infective agent comprises terbinafine.

12. The use according to claim 9, wherein the infection is onychomycosis.

## Patentansprüche

1. Feste Zusammensetzung zur Verwendung beim Behandeln von Onychomykose durch subunguale Implantation der festen Zusammensetzung in ein subunguales Nagelbett in einem Bereich, der proximal von der Lunula, lateral von den Nagelfalten und distal von der distalen Spitze des Fingers oder der Zehe begrenzt ist, wobei die feste Zusammensetzung ein oder mehrere feste Polymerimplantate mit verzögerter Wirkstofffreigabe umfasst, wobei das eine oder die mehreren festen Polymerimplantate mit verzögerter Wirkstofffreigabe zwischen etwa 30-90 Gew.-% eines Wirkstoffs umfassen, wobei die feste Zusammensetzung durch Einfügen einer Implantationsvorrichtung durch Haut oder Gewebe an der distalen Spitze eines Fingers oder einer Zehe gemäß einer vorbestimmten therapeutischen Behandlung, umfassend vorbestimmte Implantationsintervalle, subungual implantiert wird.

2. Zusammensetzung zur Verwendung nach Anspruch 1, wobei das Implantationsintervall aus der Gruppe, bestehend aus etwa 14 Tagen, etwa 30 Tagen, etwa 45 Tagen, etwa 60 Tagen, etwa drei Monaten, etwa sechs Monaten und etwa einem Jahr, ausgewählt ist.

3. Zusammensetzung zur Verwendung nach Anspruch 1 oder 2, wobei der Wirkstoff Terbinafin umfasst.

4. Zusammensetzung zur Verwendung nach Anspruch 1, wobei die eine oder die mehreren Zusammensetzungen mit verzögerter Wirkstofffreigabe ein biologisch abbaubares Polymer umfassen.

5. Zusammensetzung zur Verwendung nach Anspruch 4, wobei das biologisch abbaubare Polymer ein Poly(Milchsäure-Co-Glykolsäure) (PLGA)-Copolymer umfasst.

6. Zusammensetzung zur Verwendung nach Anspruch 4, wobei das biologisch abbaubare Polymer Polyethylenglykol umfasst.

7. Zusammensetzung zur Verwendung nach Anspruch 1, wobei die vorbestimmte therapeutische Behandlung eine kontinuierliche Behandlung oder eine gepulste Behandlung ist.

8. Zusammensetzung zur Verwendung nach Anspruch 7, wobei die gepulste Behandlung ein oder mehrere behandlungsfreie Intervalle von mindestens zwei Wochen umfasst.

9. Verwendung einer festen Zusammensetzung zur Herstellung eines Medikaments zur Verwendung beim Behandeln einer Infektion der Nageleinheit durch subunguale Implantation der festen Zusammensetzung in ein subunguales Nagelbett in einem Bereich, der proximal von der Lunula, lateral von den Nagelfalten und distal von der distalen Spitze des Fingers oder der Zehe begrenzt ist, wobei die Zusammensetzung eine oder mehrere Polymerzusammensetzungen umfasst, die aus zwischen etwa 30-90 Gew.-% eines Anti-Infektionsmittels besteht.

10. Verwendung nach Anspruch 9, wobei die eine oder die mehreren Zusammensetzungen ein Volumen zwischen 0,1 µl und 50 µl haben.

11. Verwendung nach Anspruch 9, wobei das Anti-Infektionsmittel Terbinafin umfasst.

12. Verwendung nach Anspruch 9, wobei die Infektion Onychomykose ist.

## Revendications

1. Composition solide destinée à une utilisation dans le traitement de l'onychomycose par implantation sous-unguéale de la composition solide dans le lit sous-unguéal dans une zone délimitée proximalement par la lunule, latéralement par les sillons latéraux de l'ongle et distalement par l'extrémité distale du doigt, la composition solide comprenant un ou plusieurs implants polymères solides à libération prolongée, lesdits un ou plusieurs implants polymères solides à libération prolongée comprenant entre environ 30 et 90 % en poids d'un agent actif, ladite composition solide étant implantée de manière sous-unguéale par introduction d'un dispositif d'implantation à travers la peau ou les tissus au niveau de l'extrémité distale d'un doigt conformément à un régime thérapeutique prédéterminé comprenant des intervalles d'implantation prédéterminés.

2. Composition destinée à une utilisation selon la revendication 1, dans laquelle l'intervalle d'implantation est choisi dans le groupe constitué par : environ 14 jours, environ 30 jours, environ 45 jours, environ 60 jours, environ trois mois, environ six mois et environ un an.

3. Composition destinée à une utilisation selon la revendication 1 ou la revendication 2, dans laquelle l'agent actif comprend la terbinafine.

4. Composition destinée à une utilisation selon la revendication 1, dans laquelle les une ou plusieurs compositions à libération prolongée comprennent un polymère biodégradable.

5. Composition destinée à une utilisation selon la revendication 4, dans laquelle le polymère biodégradable comprend un copolymère acide polylactique-co-glycolique (PLGA).

6. Composition destinée à une utilisation selon la revendication 4, dans laquelle le polymère biodégradable comprend le polyéthylène glycol.

7. Composition destinée à une utilisation selon la revendication 1, dans laquelle le régime thérapeutique prédéterminé est un régime continu ou un régime périodique.

8. Composition destinée à une utilisation selon la revendication 7, dans laquelle le régime périodique comprend un ou plusieurs intervalles exempts de traitement d'au moins deux semaines.

9. Utilisation d'une composition solide pour la fabrication d'un médicament destiné à une utilisation dans le traitement d'une infection de l'unité unguéale par implantation sous-unguéale de la composition solide dans le lit sous-unguéal dans une zone délimitée proximalement par la lunule, latéralement par les sillons latéraux de l'ongle et distalement par l'extrémité distale du doigt, la composition comprenant une ou plusieurs compositions polymères solides comprenant entre environ 30 et 90 % en poids d'un agent anti-infectieux.

10. Utilisation selon la revendication 9, dans laquelle les une ou plusieurs compositions ont un volume compris entre 0,1 µl et 50 µl.

11. Utilisation selon la revendication 9, dans laquelle l'agent anti-infectieux comprend la terbinafine.

12. Utilisation selon la revendication 9, dans laquelle l'infection est l'onychomycose.
